Europäisches Patentamt

European Patent Office

Office européen des brevets

(11) Veröffentlichungsnummer: **0 176 901**
**A2**

(12) **EUROPÄISCHE PATENTANMELDUNG**

(21) Anmeldenummer: **85111994.1**

(22) Anmeldetag: **21.09.85**

(51) Int. Cl.⁴: **C 12 N 9/04**
**//(C12N9/04, C12R1:26)**

(30) Priorität: **04.10.84 DE 3436350**
**28.06.85 DE 3523089**

(43) Veröffentlichungstag der Anmeldung:
**09.04.86 Patentblatt 86/15**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Stolp, Heinz, Prof. Dr.**
**Elbering 7**
**D-8580 Bayreuth(DE)**

(72) Erfinder: **Roitsch, Thomas**
**An der Kreuzkirche 59**
**D-8489 Eschenbach(DE)**

(54) Alkoholdehydrogenase, Verfahren und Stamm zu ihrer Herstellung.

(57) Aus dem methylotrophen Bakterium DSM 3031 läßt sich leicht in hoher Ausbeute eine Alkoholdehydrogenase gewinnen, die durch einen isoelektrischen Punkt von etwa 9, 4 gekennzeichnet ist. Überraschenderweise wird durch Verlängerung der Verdopplungszeit eine Überproduktion des Enzyms bewirkt.

Croydon Printing Company Ltd.

HOECHST AKTIENGESELLSCHAFT    HOE 85/F 114 J   Dr.Kh/je

Alkoholdehydrogenase, Verfahren und Stamm zu ihrer
Herstellung

Die Erfindung betrifft eine neue Alkoholdehydrogenase,
die durch einen isoelektrischen Punkt von etwa 9,4 gekennzeichnet ist. Trotz dieses hohen isoelektrischen
Punkts überwiegen in der Aminosäurenzusammensetzung die
basischen Aminosäuren nicht. Der Glycingehalt liegt etwa
doppelt so hoch wie bei bekannten Alkoholdehydrogenasen.

Die erfindungsgemäße Alkoholdehydrogenase wird aus
Zellen eines methylotrophen Mikroorganismus isoliert, der
bei der DSM unter der Nummer 3031 hinterlegt ist und für
den die taxonomische Bezeichnung Methanolomonas glucoseoxydans vorgeschlagen wurde. Dieser Stamm wurde aus einem
Schlamm isoliert. Er wächst auf Methanol oder Glucose als
Kohlenstoffquellen, oxidiert Methan nicht und verwertet
weder andere Zucker noch andere Alkohole und auch keine
organischen Säuren einschließlich der Aminosäuren und
verwertet auch nicht komplexe Medien. In einem Methanolmedium beträgt die Verdoppelungsrate $t_d$ 2 Stunden, in
einem Glucosemedium 12 Stunden.

Der Mikroorganismus ist aerob und gram-negativ und bildet
einzelne Stäbchen (0,5 x 0,95 µm), die mit einer polaren
Geißel beweglich sind. Der Stamm zeigt keine Pigmentierung und bildet keine Sporen. Die Tests auf Katalase und
Oxidase sind positiv.

Als Stickstoffquellen verwertet der Stamm Ammonium, Nitrat oder methylierte Amine.

Die in dem Mikroorganismus gebildete Alkoholdehydrogenase ist in ihrer Wirkung von der Gegenwart von Ammoniumionen abhängig. Sie ist bei hohen pH-Werten (pH 9,0)
aktiv und wird von 1 mM Cyanid stimuliert.

In Zellen, die auf Methanol in "batch"-Kultur mit einer
Verdopplungszeit von 2 Stunden wachsen, beträgt der Anteil
der Alkoholdehydrogenase 8,4 % des löslichen Proteins.
Überraschenderweise hat sich gezeigt, daß durch eine
Reduzierung der Wachstumsrate auf eine verlängerte
Verdopplungszeit von 8 bis 14 Stunden, vorzugweise 10 bis
12 Stunden, eine Überproduktion des Enzyms auf 20 bis 30 %
des löslichen Proteins erreicht wird. Dies geschieht bevorzugt durch "batch"-Kultivierung auf Glucose als Kohlenstoffquelle und/oder bei Wachstum auf Methanol durch
Limitierung dieser C-Quelle, beispielweise in kontinuierlicher Kultur. Diese Erscheinung ist ungewöhnlich, da
eine Überproduktion für das Wachstum unter methanollimitierten Bedingungen und insbesondere für das Wachstum und
den Metabolismus auf Glucose für die Zelle unnötig ist.
Bei den bisher bekannten fakultativ methylotrophen Mikroorganismen wird die Synthese des Enzymes unter den genannten Bedingungen entsprechend unterdrückt.

Die erfindungsgemäße Alkoholdehydrogenase läßt sich besonders vorteilhaft durch Chromatographie des Rohproteins
an Kationenaustauschern isolieren. Hierfür können die
handelsüblichen kationisierten Kunstharze verwendet werden, die unter den Handelsmarken LEWATIT, PERMUTIT,
AMBERLITE und DOWEX im Handel sind, besonders aber kationisch modifizierte Polysaccharid-Derivate, wie sie beispielsweise unter den Handelsmarken CM und SP SEPHAROSE
bekannt sind.

Das durch Zellaufschluß gewonnene und in einem geeigneten
Puffer gelöste Rohprotein wird auf den Kationenaustauscher gegeben und die übrigen Proteine durch Elution
mit der Pufferlösung entfernt. Anschließend kann das basische Enzym mittels eines Salzgradienten von dem Sorbens eluiert werden. Als Salze für den Salzgradienten eignen sich leicht lösliche Salze, vorzugsweise Halogenide, Sulfate oder Nitrate der Alkali- bzw. Erdalkalimetalle in Konzentrationen von 0,01 bis 1 Mol pro Liter. Das Eluat wird fraktioniert gesammelt. Durch Proteinbestimmung in den Einzelfraktionen bzw. durch UV-Detektion kann das Elutionsdiagramm der Chromatographie bestimmt werden.

In den folgenden Beispielen wird die Erfindung näher erläutert. Prozentangaben beziehen sich auf das Gewicht, wenn nichts anderes angegeben ist.

Folgende analytischen Methoden wurden zur Quantifizierung und Charakterisierung der Alkoholdehydrogenase herangezogen:

1. Enzym-Assay: Methode für farbstoffgebende Alkoholdehydrogenasen (EC 1.1.99.8) (C.W. Bamforth et al., Biochem. J. 169, 677-686 (1978)).

2. Bestimmung der Michaelis-Konstanten $K_m$: Messungen im Enzym-Assay bei unterschiedlichen Substrat-(Methanol)-Konzentrationen (H. Lineweaver et al., J. Am. Chem. Soc. 56, 658-666 (1934).

3. Enzymaktivität: Reduktion von 2,6-Dichlorphenolindophenol (R.E. Basford, J.Am.Chem. Soc. 77, 3873-3877 (1955)).

4. Protein-Bestimmung: Methode nach Bradford (M.M. Bradford, Anal. Biochem. 72, 248-254 (1976)).

5. Gesamtstickstoff: Methode nach Kjeldahl.

6. Aminosäureanalyse: Hydrolyse bei 115°C, 6 n HCl, Analyse durch Ionenaustauschchromatographie.

7. SDS-Gelelektrophorese (U.K. Laemmli, Nature 227, 680-685 (1970)).

8. Chromatographie an DEAE-Cellulose.

Beispiel 1

300 ml eines Mineralsalz-Mediums der Zusammensetzung

2,72  g/l $KH_2PO_4$;

0,54  g/l $NH_4Cl$;

0,2   g/l $MgSO_4$ x $7H_2O$;

0,015 g/l $CaCl_2$ x $2H_2O$;

0,003 g/l $FeCl_3$ x $6H_2O$ und

1 ml Spurenelemente-Lösung

0,05  g/l $H_3BO_3$

0,01  g/l KJ

0,04  g/l $MnSO_4$ x 4 $H_2O$

0,04  g/l $ZnSO_4$ x 7 $H_2O$

0,02  g/l $(NH_4)_6Mo_7O_{24}$

wird mit 1 N NaOH auf pH 6,8 eingestellt und autoklaviert. Nach Ergänzung mit 0,5 % (v/v) sterilfiltriertem Methanol wird mit dem Stamm DSM 3031 angeimpft und 1 Tag bei 30°C im Erlenmeyerkolben geschüttelt. Die Zellsuspension wird abzentrifugiert und nach Resuspension mit kaltem Tris-HCl-Puffer (pH = 7,5) in einer Frenchpresse homogenisiert und durch Zentrifugation von Zellwandfragmenten befreit. Durch Gefriertrocknen werden 1,3 g eines rohen Proteinextraktes gewonnen, dessen Proteingehalt zu 82 % bestimmt wurde. Durch SDS-Elektrophorese wird die Proteinzusammensetzung bestimmt.

1,3 g des 82%igen Rohproteins (1,07 g Gesamtprotein) wird in 10 ml 20 mM Tris-HCl-Puffer (pH 8,0) gelöst und auf eine mit dem gleichen Puffer äquilibrierte Säule, gefüllt mit CM-Sepharose, aufgetragen. Die Fremdproteine werden mit dem gleichen Puffer ausgewaschen und anschließend die Alkoholdehydrogenase mit einem KCl-Gradienten von 0,02-0,6 mol/l eluiert. Die Homogenität der isolierten Alkoholdehydrogenase wird durch SDS-Elektrophorese sichergestellt. Nach Dialyse gegen salzfreies Wasser und Gefriertrocknung erhält man 268 mg (25 % des Gesamtproteins) der reinen Alkoholdehydrogenase.

Das Ergebnis zeigt die Tabelle 1.

Beispiel 2

Das Experiment wird analog Beispiel 1 durchgeführt, wobei aber anstatt Methanol als C-Substrat Glucose in einer Konzentration von 50 mM zugegeben und 3 Tage bei 30°C geschüttelt wird. Die Aufarbeitung erfolgt analog Beispiel 1 oder wie folgt:

Man benützt anstatt der CM-Sepharose einen Kunstharz-Ionenaustauscher vom Typ Dowex CCR-2 (K-Form) und erhält die reine Alkoholdehydrogenase in einer Ausbeute von 22 % des Gesamtproteins.

Das Ergebnis zeigt Tabelle 1.

Tabelle 1: Vergleich der Alkoholdehydrogenase in Extrakten von glucose- und methanolgewachsenen
Zellen vom Stamm DSM 3031

| Alkoholdehydrogenase | Aktivität oder Proteinmenge | | Verhältnis-zahl |
| --- | --- | --- | --- |
| | Glucose-Substrat | Methanol-Substrat | |
| Spezifische Aktivität (mU/mg lösl. Protein) | 1 692 | 535 | 3,2 |
| Spezifische Aktivität (mU/mg Gesamt-N) | 10 263 | 2 471 | 4,2 |
| % Enzym-Protein, bezogen auf lösl. Protein aufgrund DEAE-Chromat. | 25,0 | 8,4 | 3,0 |
| aufgrund spez. Aktivität | 22,8 | 8,4 | 2,7 |

Vergleich der aus Beispiel 1 und Beispiel 2 gewonnenen
Alkoholdehydrogenase:

1. Michaelis-Konstante ($K_m$):

Die spezifische Aktivität der Alkoholdehydrogenasen
aus glucose- und methanolgewachsenen Zellen wurde für
beide zu $3,8 \times 10^{-5}$ bestimmt. Dieser Befund wird als
Beweis dafür gewertet, daß in den auf Glucose gewachsenen Zellen eine größere Mengen desselben Enzyms
(und nicht etwa ein aktiveres anderes) gebildet wird.

2. SDS-Elektrophorese

Ein Vergleich der durch DEAE-Cellulose-Chromatographie gereinigten Alkoholdehydrogenase aus Beispiel 1 und 2 zeigt
identische Molgewichte.

3. Aminosäure-Analyse:

Die Aminosäureanalyse zeigt folgende identische Zusammensetzung der gereinigten Enzyme (mMol Aminosäure pro 100 mg Protein):

Ala 104, Arg 19, Asp 99, Cys (1/2) 10, Glu 101, Gly 177, His 18, Ile 35, Leu 105, Lys 77, Met 30, Phe 33, Pro 67, Ser 49, Thr 51, Tyr 51, Val 85.


Beispiel 3

Man kultiviert den Stamm DSM 3031 unter den in Beispiel 1 aufgeführten Bedingungen in kontinuierlicher Kultur mit einer Verdünnungsrate von 0,0577 $h^{-1}$, die einer Verdopplungszeit des Mikroorganismus von 12 Stunden entspricht. Die Aufarbeitung erfolgt analog Beispiel 1 oder Beispiel 2. Es werden die gleichen Ergebnisse wie in Beispiel 2 erreicht.

PATENTANSPRÜCHE:

1. Alkoholdehydrogenase, gekennzeichnet durch einen isoelektrischen Punkt von etwa 9,4.

2. Verfahren zur Herstellung einer Alkoholdehydrogenase nach Anspruch 1, dadurch gekennzeichnet, daß man den Mikroorganismenstamm DSM 3031 kultiviert und aus den Zellen die gebildete Alkoholdehydrogenase gewinnt.

3. Verfahren nach Anspruch 2, dadurch gekennzeichnet, daß der Mikroorganismenstamm DSM 3031 unter Bedingungen, die eine verlängerte Verdopplungszeit bewirken, kultiviert wird.

4. Verfahren nach Anspruch 3, dadurch gekennzeichnet, daß die Verdopplungszeit 8 bis 14 Stunden beträgt.

5. Verfahren nach Anspruch 4, dadurch gekennzeichnet, daß die Verdopplungszeit 10 bis 12 Stunden beträgt.

6. Verfahren nach Anspruch 3 bis 5, dadurch gekennzeichnet, daß der Mikroorganismus DSM 3031 auf Methanol unter Limitierung dieser Kohlenstoffquelle kultiviert wird.

7. Verfahren nach Anspruch 3 bis 5, dadurch gekennzeichnet, daß der Mikroorganismus DSM 3031 auf Glucose als Kohlenstoffquelle kultiviert wird.

8. Verfahren nach Anspruch 2 bis 7, dadurch gekennzeichnet, daß man die Alkoholdehydrogenase durch Chromatographie an einem Kationenaustauscher isoliert.

9. Mikroorganismenstamm DSM 3031.

PATENTANSPRÜCHE ÖSTERREICH:

1. Verfahren zur Herstellung einer Alkoholdehydrogenase mit einem isoelektrischen Punkt von etwa 9,4, dadurch gekennzeichnet, daß man den Mikroorganismenstamm DSM 3031 kultiviert und aus den Zellen die gebildete Alkoholdehydrogenase gewinnt.

2. Verfahren nach Anspruch 1, dadurch gekennzeichnet, daß der Mikroorganismenstamm DSM 3031 unter Bedingungen, die eine verlängerte Verdopplungszeit bewirken, kultiviert wird.

3. Verfahren nach Anspruch 2, dadurch gekennzeichnet, daß die Verdopplungszeit 8 bis 14 Stunden beträgt.

4. Verfahren nach Anspruch 3, dadurch gekennzeichnet, daß die Verdopplungszeit 10 bis 12 Stunden beträgt.

5. Verfahren nach Anspruch 2 bis 4, dadurch gekennzeichnet, daß der Mikroorganismus DSM 3031 auf Methanol unter Limitierung dieser Kohlenstoffquelle kultiviert wird.

6. Verfahren nach Anspruch 2 bis 4, dadurch gekennzeichnet, daß der Mikroorganismus DSM 3031 auf Glucose als Kohlenstoffquelle kultiviert wird.

7. Verfahren nach Anspruch 1 bis 6, dadurch gekennzeichnet, daß man die Alkoholdehydrogenase durch Chromatographie an einem Kationenaustauscher isoliert.